# EUROPÄISCHE PATENTSCHRIFT

(11) **EP 1 270 079 B1**
(45) Veröffentlichungstag und Bekanntmachung des Hinweises auf die Patenterteilung: **07.12.2005**
(21) Anmeldenummer: 02013724.6
(22) Anmeldetag: 20.06.2002
(51) Int. Cl.: B04B 5/04

(54) **Schlauchanordnung und Verfahren zu ihrer Herstellung**
Tube arrangement and its manufacturing method
Arrangement de tuyau et son procédé de fabrication

(30) Priorität: 20.06.2001 DE 10129769
(43) Veröffentlichungstag der Anmeldung: 02.01.2003
(73) Patentinhaber: Fresenius HemoCare GmbH, 61352 Bad Homburg v.d.H. (DE)
(72) Erfinder: Witthaus, Friedrich, 66640 Namborn (DE); Weber, Wolfram, 66583 Spiesen-Elversberg (DE)
(74) Vertreter: Laufhütte, Dieter

(56) Entgegenhaltungen:
- WO-A-01/30505
- DE-A- 3 632 241
- DE-A- 19 803 535

## Beschreibung

Die Erfindung betrifft eine mehrlumige Schlauchanordnung zum Einsatz in einer gleitdichtungsfreien Zentrifuge nach dem Oberbegriff des Anspruchs 1.

Im Stand der Technik sind gleitdichtungsfreie Zentrifugen beispielsweise aus der DE 32 42 541 oder DE 42 20 232 bekannt. Dabei wird ein Stoff per Zentrifugalkraft in seine Einzelteile zerlegt und über Schläuche in separate Behälter befördert. Da die Behälter ortsfest sind, aber die Trenneinheit sich dreht, sind verschiedene Lösungen bekannt, wie die Schlauchverbindung zwischen beiden zu führen ist. Möchte man zur Vermeidung von Kontamination auf eine Gleitdichtung verzichten, bietet sich die Verwendung oben genannter Zentrifugen an.

Wie in der DE 26 12 988 dargestellt, kann der Multilumenschlauch aus einem einzigen Schlauch mit mehreren Flüssigkeitskanälen bestehen, während die DE 36 32 241 eine Schlauchanordnung aus mehreren Einzelschläuchen zeigt. Die Schläuche oder die mehrlumige Schlauchanordnung werden bei einer gleitdichtungsfreien Zentrifuge von einer ortsfesten Anschlußstelle in einer Schleife um die Trenneinheit herumgeführt zur gegenüberliegenden Seite der Trenneinheit.

Während des Betriebs der gleitdichtungsfreien Zentrifuge wird die Schlauchanordnung mit einer Antriebseinheit verbunden, die sich mit halber Winkelgeschwindigkeit bzw. Drehzahl dreht wie die Trenneinheit, des weiteren dreht sich die Schlauchanordnung um sich selbst. Infolge der unterschiedlichen Drehzahlen zwischen Antriebseinheit und Trenneinheit entzwirnt sich die Schlauchanordnung.

Dabei kommt die radial weiter außenliegende Schlauchseite auf die Innenseite und umgekehrt, so daß die ganze Schlauchanordnung gewalkt wird. Die außenliegende Schlauchseite wird gestreckt, während die innenliegende gestaucht wird, da die Wege von dem ortsfesten Anschluß bis zur Trenneinheit unterschiedlich lang sind. Aus diesem Grund wird in der DE 36 32 241, auf die hier ausdrücklich Bezug genommen werden soll, vorgeschlagen, einzelne Schläuche oder Schlauchlumen n+1/2 mal um die Schlauchlängsachse bzw. deren Parallelachsen zu winden, um die Wegstrecken im Mittel gleich zu gestalten.

Darüber hinaus unterliegt die Schlauchanordnung wegen der Zentrifugalkräfte einer hohen mechanischen Beanspruchung, die mit zunehmender Drehzahl steigt. Die Leitung bildet eine nach außen ausladende Schleife, die an ihren Enden hohen Biegekräften bzw. Abriebkräften ausgesetzt ist. Aus diesem Grund sind die Schläuche in gleitdichtungsfreien Zentrifugen häufig gelagert, wie dies in der DE 198 03 534 beschrieben ist. Diese Lager können Rollenlager sein wie in der WO 95/17261 oder Gleitlager wie in der EP 0 112 990.

Bei einer Schlauchanordnung einer gleitdichtungsfreien Zentrifuge ergibt sich an der Lagerstelle aufgrund der Lagerreibung ein Drehmoment, welches von beiden Schlauchenden auf die Lagerstelle übertragen werden muß. Dieses Drehmoment erzeugt eine zusätzliche Belastung des Schlauches, in dem es der Torsionssteifigkeit des Schlauches und damit der Lebensdauer entgegenwirkt. Aus der WO-A-0130505 ist bereits eine mehrlumige Schlauchanordnung nach dem Oberbegriff des Anspruchs 1 bekannt.

Es ist Aufgabe der vorliegenden Erfindung, eine Schlauchanordnung der eingangs erwähnten Art derart fortzubilden, daß die Lebensdauer weiter deutlich erhöht wird und sie dennoch eine kostengünstige Herstellung erlaubt. Die Lösung der Aufgabe erfolgt mit den in Anspruch 1 aufgezeigten Merkmalen.

Die erfindungsgemäße Schlauchanordnung besteht aus mindestens zwei Einzelschläuchen, die um ihre Längsachse oder deren Parallelachse umeinander gedreht sind und an ihren Enden fixiert sind und die eine Lagerstelle aufweist, von der aus die Schläuche in entgegengesetzter Richtung bis zu ihren Enden umeinander gedreht sind.

Aufgrund der gewünschten Entzwimung der Schlauchanordnung ist das Drehmoment, welches von der Trenneinheit zum Lager übertragen wird entgegengesetzt denjenigen von der ortsfesten Stelle zum Lager. Es wurde festgestellt, daß die Standfestigkeit oder Lebensdauer der Schlauchanordnung darunter leidet, wenn das übertragene Drehmoment in entgegengesetzter Richtung zur Schlauchverdrehung wirkt. Daher wurden Versuche durchgeführt, die die Lebensdauer der Schlauchanordnung in Abhängigkeit der Verdrehrichtung der beiden Schlauchenden vom Lager aus mißt. Die Versuche belegen eine wesentlich erhöhte Standfestigkeit, wenn das auf die Schlauchanordnung einwirkende Drehmoment in gleicher Richtung verläuft wie die Verdrehung der Einzelschläuche umeinander jeweils ausgehend von der Lagerstelle zu ihren fixierten beiden Enden. Da, wie oben beschrieben, jedoch die Drehmomente entgegengesetzt wirken, ist die Verdrehung der Schläuche um ihre Längsachse bzw. um deren Parallelachse von der Lagerstelle fortführend erfindungsgemäß ebenfalls in entgegengesetzter Richtung.

Die Schlauchanordnung kann, wie im Stand der Technik beschrieben, aus einem Schlauch mit mehreren Lumen, die dann ebenfalls um ihre Längsachse gewunden sind, bestehen oder aus mehreren Einzelschläuchen. Der Einfachheit halber wird vorliegend von mehrlumiger Schlauchanordnung mit mindestens zwei Einzelschläuchen gesprochen, wobei aber auch ein Multilumenschlauch mit mehr als zwei Lumen zu subsumieren ist, wobei dann die Einzellumen gemäß der Erfindung in entgegengesetzter spiraliger Form von der Lagerstelle aus verlaufen.

Die erfindungsgemäß eingesetzten Schläuche bestehen aus einem polymeren Material aus ein oder mehreren Schichten, wie Polyamid, Polyurethan oder PVC, sowie insbesondere Polyolefine wie Polyethylene oder Polypropylene und deren Blends, wobei in unterschiedlichen Schichten unterschiedliche Materialien eingesetzt werden können.

Wenn die Schläuche im medizinischen Bereich eingesetzt werden, sind biokompatible Materialien natürlich besser geeignet. Während in der Vergangenheit PVC-Schläuche gängig waren, sind mittlerweile Polyolefinschläuche gebräuchlich, bevorzugt mehrschichtige Poleolefinschläuche, deren Härte in jeder Schicht durch Blends auch mit Ethylenstyrenen eingestellt werden kann.

Durch geeignete Wahl der Polymermaterialien und der eingesetzten Schichten - sei es in Form von extrudierten Schläuchen oder laminierten Schläuchen - weist die erfindungsgemäße Schlauchanordnung eine hohe Festigkeit auf, gegenüber Längung, Knicken oder Abdrehen. Auch der Innendurchmesser kann im wesentlichen über die Gesamtlänge konstant gehalten werden, so daß keine Gefahr des Verstopfens besteht.

Üblicherweise besteht eine derartige Schlauchanordnung aus zwei bis fünf Lumen oder Einzelschläuchen, vorzugsweise vier. Die Einzelschläuche werden zumindest in Teilbereichen um die Längsachse der Schlauchanordung oder um eine parallele Achse hierzu herumgedreht, so daß ein in sich verdrehtes Bündel entsteht. Das Verdrehen erfolgt dabei nach üblichen Methoden des Verseilens oder Flechtens.

Dabei ist die Schlauchanordnung beispielsweise durch Klemmen oder Verkleben an der Lagerstelle fixiert, während die Enden sich an Haltestücken befinden, deren Aufnahmebohrungen die Bewegung der Einzelschläuche noch erlaubt, also wenig größer sind als der Schlauchdurchmesser, so daß eine Entdrillung stattfinden kann.

Dadurch stehen die Einzelschläuche nicht unter zusätzlicher Spannung, die die Lebensdauer der Schläuche ebenfalls herabsetzt. Vorzugsweise erfolgt dabei die Verdrehung n+1/2-fach, wobei n null oder eine ganze Zahl ist. Besonders bevorzugt werden die Einzelschläuche 1,5fach gedreht.

Dabei werden die Einzelschläuche an der Lagerstelle fixiert und die Haltestücke, in denen die Einzelschläuche zunächst lose gehalten sind, gegen die Lagerstelle verschoben, verdreht und wieder in ihre Ausgangsposition zurückgeschoben. Danach werden die Enden fixiert, sei es durch Einklemmen in das Haltestück, Verkleben im Haltestück oder Verkleben der Einzelschläuche untereinander.

Das Herstellungsverfahren ist dabei nicht auf die vorstehend beschriebene Verfahrensweise beschränkt, sondern kann selbstverständlich auch erfolgen, indem die Enden bereits fixiert werden, während die Lagerstelle gegen das Haltestück geschoben wird, gedreht wird und wieder in seine Ausgangsposition zurückgeschoben wird. Dabei muß das Lager Bohrungen aufweisen, die so groß sind, daß sie eine Eigenbewegung der Schläuche erlauben und die Verdrillung der Einzelschläuche um sich selbst vermeiden. Da durch das Drehen der Lagerstelle nur eine Bewegung erfolgen muß, mit der gleichzeitig beide Schlauchteile in entgegengesetzte Richtungen verdreht werden, ist dieses einfachere Herstellungsverfahren bevorzugt.

Weitere Einzelheiten und Vorteile der Erfindung ergeben sich aus einem in der Zeichnung dargestellten Ausführungsbeispiel:
- Fig. 1:: zeigt eine Schlauchanordnung in einer gleitdichtungsfreien Zentrifuge.
- Fig. 2:: zeigt die ausgestreckte Schlauchanordnung mit ausgehend von der Lagerstelle in entgegengesetzter Richtung verdrehten Einzeischläuchen.

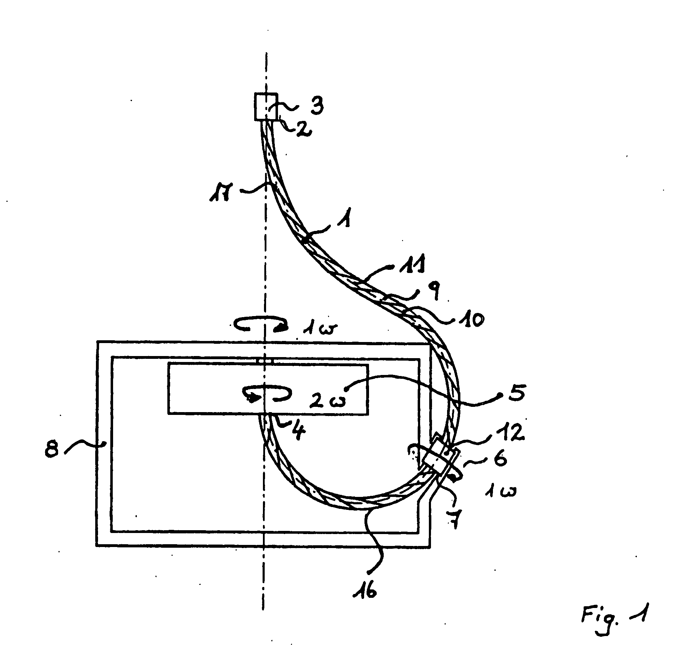

In Figur 1 ist die Schlauchführung in einer gleitdichtungsfreien Zentrifuge schematisch gezeigt. Dabei ist die Schlauchanordnung 1 an ihrem ersten Ende 2 an einer ortsfesten Stelle 3 fixiert sowie an ihrem zweiten Ende 4 einer Trenneinheit 5 befestigt. Die Schlauchanordnung wird an einer Lagerstelle 6 mittels einer Lagerhalterung 12 eines Gleitlagers 7 gehalten, welches seinerseits in einem Drehrahmen 8 gelagert ist. Wenn sich die Trenneinheit 5 mit einer Winkelgeschwindigkeit 2ω dreht, wird die an der Stelle 4 an dieser Trenneinheit befestigte Schlauchanordnung mitgedreht. Gleichzeitig führt der Drehrahmen 8 die Schlauchanordnung 1 um die Trenneinheit 5 herum, indem sich der ganze Drehrahmen 8 mit einer Winkelgeschwindigkeit von 1ω in entgegengesetzter Richtung zur Trenneinheit 5 dreht. Die Schlauchanordnung 1 dreht sich mit ebenfalls 1ω um sich selbst, so daß sich die Summe der Winkelgeschwindigkeiten der Schlauchführung zu der der Trenneinheit aufheben und daher keine Verdrillung bzw. kein Abscheren beispielweise an der ortsfesten Fixierung 2 erfolgt.

Durch die Drehung der Trenneinheit 5 wird ein Drehmoment auf die Schlauchanordnung 1 übertragen, das dem Drehmoment, welches von der ortsfesten Fixierung 2 aus auf die Schlauchanordnung übertragen wird, entgegengesetzt wirkt, da die Schlauchanordnung quasi u-förmig verläuft. Um die Verdrehung der Einzelschläuche 9 und 10 nicht dem Drehmoment entgegenwirken zu lassen, sind die Einzelschläuche 9, 10 in den Teilabschnitten 16, 17 der Schlauchanordnung jeweils von der Lagerstelle 6 ausgehend zu ihren Enden 2 und 4 hin gesehen in der gleichen Richtung um ihre Lächsachse 11 bzw. deren Paralleiachse gewunden, geflochten oder gedreht wie die Wirkung des Drehmoments.

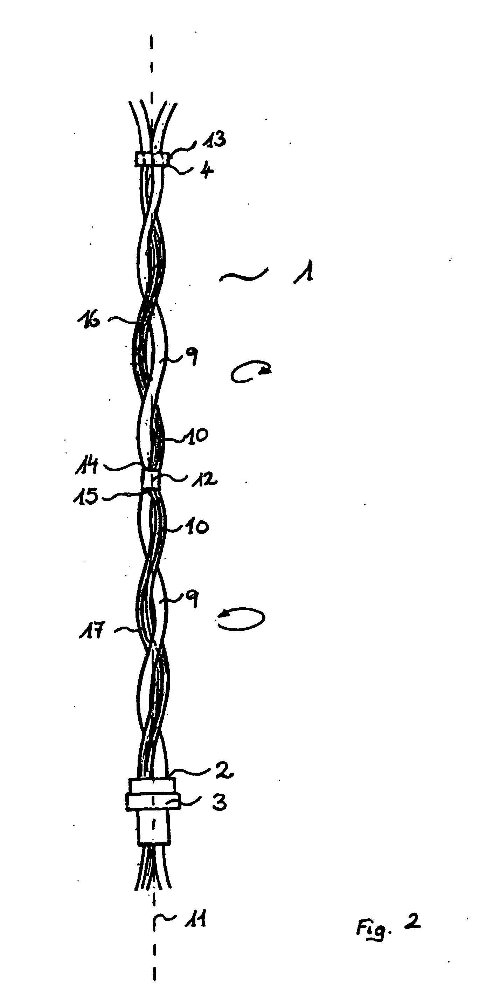

Figur 2 zeigt die Schlauchanordnung 1 in ausgestrecktem Zustand. Die Einzelschläuche 9 und 10 sind von der Lagerstelle 12 ausgehend in gegenläufiger Richtung zu ihren Enden 2 und 4 um ihre Längsachse 11 gedreht. Dabei sind die Enden in einer Halterung 3 bzw. 13 fixiert sowie in ihrer Lagerhalterung 12, so daß keine Entdrehung stattfinden kann. Dabei sind die Einzelschläuche vorzugsweise in n+1/2 Drehungen umeinander gewunden, wobei n 0 oder eine ganze Zahl ist, so daß die Enden der Einzelschläuche 4, 2 jeweils an der um 180° versetzten Seite zu ihrem an der Lagerhalterung 12 befestigen Ende 14 und 15 zu liegen kommen. Dadurch besitzt jeder Einzelschlauch oder jedes Lumen in etwa die gleiche Länge.

Vorzugsweise weist jedes Teilstück 16, 17 der Schlauchanordnung 1 eine anderthalbfache Drehung auf.

## Patentansprüche

1. Mehrlumige Schlauchanordnung (1) zum Einsatz in einer gleitdichtungsfreien Zentrifuge mit mindestens zwei, etwa gleichlangen Einzelschläuchen oder einem Schlauch mit mehreren Lumen, wobei die Einzelschläuche oder die lumen um die Längsachse (11) der Schlauchanordnung (1) oder deren Parallelachse spannungsfrei umeinander gewunden sind und an ihren Enden (2, 4) fixiert sind,
**dadurch gekennzeichnet,**
**daß** die Schlauchanordnung(1) eine Lagerstelle (6) aufweist und daß die Einzelschläuche oder die lumen von der Lagerstelle (6) aus gehend zu ihren Enden hin in entgegengesetzter Richtung umeinander gewunden und an der Lagerstelle (6) fixiert sind.

2. Schlauchanordnung nach Anspruch 1 **dadurch gekennzeichnet, daß** die einen Enden der Schläuche (1) gegenüber der Lagerstelle (6) um n+1/2 Drehungen verdreht sind, wobei n eine 0 oder eine ganze Zahl ist.

3. Schlauchanordnung nach einem der vorhergehenden Ansprüche **dadurch gekennzeichnet, daß** die Verdrehung etwa 1,5 ist.

4. Schlauchanordnung nach einem der vorhergehenden Ansprüche **dadurch gekennzeichnet, daß** die Lagerstelle (6) als Gleitlager (7) ausgebildet ist.

5. Schlauchanordnung nach einem der Ansprüche 1 bis 3 **dadurch gekennzeichnet, daß** die Lagerstelle (6) als Rollenlager ausgebildet ist.

6. Schlauchanordnung nach einem der vorhergehenden Ansprüche **dadurch gekennzeichnet, daß** sich an den Enden (2, 4) der Schläuche zur Fixierung Haltestücke befinden.

7. Schlauchanordnung nach einem der vorhergehenden Ansprüche **dadurch gekennzeichnet, daß** die Lagerstelle (6) und/oder die Haltestücke mehrere Bohrungen zur Aufnahme der Einzelschläuche (1) aufweisen.

8. Schlauchanordnung nach einem der vorhergehenden Ansprüche, **gekennzeichnet durch** zwei bis fünf, vorzugsweise vier, Einzelschläuche (1).

## Claims

1. A multi-lumen tube arrangement (1) for use in a sliding seal-free centrifuge comprising at least two approximately equal-length individual tubes or a tube having a plurality of lumens, wherein the individual tubes or the lumens are wound in a stress-free condition around each other around the longitudinal axis (11) of the tube arrangement (1) or the parallel axis thereof and are fixed at their ends (2, 4),
**characterised in that** the tube arrangement (1) has a support location (6) and that the individual tubes or the lumens are wound around each other in opposite directions starting from the support location (6) towards their ends and are fixed at the support location (6).

2. A tube arrangement according to claim 1 **characterised in that** the one ends of the tubes (1) are rotated with respect to the support location (6) through n+1/2 turns, wherein n is 0 or an integer.

3. A tube arrangement according to one of the preceding claims **characterised in that** the rotation is about 1.5.

4. A tube arrangement according to one of the preceding claims **characterised in that** the support location (6) is in the form of a plain bearing (7).

5. A tube arrangement according to one of claims 1 to 3 **characterised in that** the support location (6) is in the form of a rolling bearing.

6. A tube arrangement according to one of the preceding claims **characterised in that** holding portions are disposed at the ends (2, 4) of the tubes for fixing purposes.

7. A tube arrangement according to one of the preceding claims **characterised in that** the support location (6) and/or the holding portions have a plurality of bores for receiving the individual tubes (1).

8. A tube arrangement according to one of the preceding claims **characterised by** two to five and preferably four individual tubes (1).

## Revendications

1. Agencement de tuyaux à plusieurs lumières (1) pour l'utilisation dans une centrifugeuse exempte de joints d'étanchéité coulissants avec au moins deux tuyaux individuels à peu près de même longueur ou un tuyau avec plusieurs lumières, où les tuyaux individuels ou les lumières sont tordus autour de l'axe longitudinal (11) de l'agencement de tuyaux (1) ou son axe parallèle sans tension les uns autour des autres et sont fixés à leurs extrémités (2, 4),
**caractérisé en ce que**
l'agencement de tuyaux (1) présente un emplacement de palier (6), et que les tuyaux individuels ou les lumières sont tordus à partir de l'emplacement de palier (6) vers leurs extrémités dans la direction opposée les uns autour des autres et sont fixés à l'emplacement de palier (6).

2. Agencement de tuyaux selon la revendication 1, **caractérisé en ce que** les extrémités des tuyaux (1) sont tordues par rapport à l'emplacement de palier (6) selon n+1/2 tours, où n représente 0 ou est un nombre entier.

3. Agencement de tuyaux selon l'une des revendications précédentes, **caractérisé en ce que** la torsion représente environ 1,5.

4. Agencement de tuyaux selon l'une des revendications précédentes, **caractérisé en ce que** l'emplacement de palier (6) est réalisé comme palier à glissement (7).

5. Agencement de tuyaux selon l'une des revendications 1 à 3, **caractérisé en ce que** l'emplacement de palier (6) est réalisé comme palier à rouleaux.

6. Agencement de tuyaux selon l'une des revendications précédentes, **caractérisé en ce que** pour la fixation, des pièces de retenue se trouvent aux extrémités (2, 4) des tuyaux.

7. Agencement de tuyaux selon l'une des revendications précédentes, **caractérisé en ce que** l'emplacement de palier (6) et/ou les pièces de retenue présentent plusieurs perçages pour la réception des tuyaux individuels (1).

8. Agencement de tuyaux selon l'une des revendications précédentes, **caractérisé par** deux jusqu'à cinq, de préférence quatre tuyaux individuels (1).
